# EUROPEAN PATENT APPLICATION

(11) **EP 2 807 993 A1**
(43) Date of publication of application: **03.12.2014**
(21) Application number: 12866422.4
(22) Date of filing: 21.11.2012
(51) Int. Cl.: A61F 9/007

(54) **LACRIMAL DUCT TUBE**

(30) Priority: 26.01.2012 JP 2012014608
(71) Applicant: Kaneka Corporation, Osaka-shi, Osaka 530-8288 (JP)
(72) Inventor: MATSUMOTO, Mariko, Settsu-shi Osaka 566-0072 (JP); MIYAUCHI, Hidekazu, Settsu-shi Osaka 566-0072 (JP)
(74) Representative: TBK
(86) International application number: PCT/JP2012/080180
(87) International publication number: WO 2013/111435

(57) **Abstract**

A lacrimal duct tube (31) includes an integrated tube that is placed in a lacrimal duct and capable of insertion of an insertion aid (36) in a detachable manner. The tube (31) has a terminal end opening (33) at a terminal end. In the vicinity of the terminal end opening (33), a stopper part (32a, 32b, 32c, and 32d) for stopping the insertion aid (36) is inserted in the tube (31). The stopper part (32a, 32b, 32c, and 32d) is opened at both ends in an axial direction of the tube and has a cylindrical structure including an inner wall surface (37a, 37b, 37c, and 37d) to guide and stop the insertion aid (36).

## Description

### Technical Field

The present invention relates to a lacrimal duct tube for use in treatment of lacrimal duct obstruction.

### Background Art

Treatment methods for lacrimal duct obstruction resulting in epiphora include: (i) probing by a lacrimal duct bougie, (ii) placement of a lacrimal duct intubation instrument; (iii) dacryocystorhinostomy (DCR); (iv) lacrimal canaliculoplasty; (v) nasolacrimal duct plasty; (vi) lacrimal caruncle moving surgery, and the like.

The probing by a lacrimal duct bougie in (i) is intended to insert a narrow tube called bougie into a lacrimal duct to open an obstructed site and reconstruct a flow path for a lacrimal fluid. This method is conducted as a first treatment in many cases due to its ease of execution and minimal invasiveness. The treatments (iii) dacryocystorhinostomy (DCR), (iv) lacrimal canaliculoplasty, (v) nasolacrimal duct plasty, (vi) lacrimal caruncle moving surgery are highly effective but relatively invasive because of the need for creation of incisions in a patient's face or drilling holes in bones, and thus are conducted as a last resort.

Lacrimal duct intubation instrument (such as a lacrimal duct tube) for use in placement of a lacrimal duct intubation instrument (ii) is, after the probing by a lacrimal duct bougie (i), placed for maintaining of a flow path and reconstruction of tissues. The placement of a lacrimal duct intubation instrument (ii) is easy, less invasive, and highly effective as compared to the foregoing treatment methods (iii) to (vi), and thus is widely performed all over the world. Among such instruments, there is widely available a so-called nunchaku-shaped lacrimal duct tube in which a central part of the tube is formed by a narrow and soft tube or rod and both sides of the tube are formed by hard and thick tubes, as disclosed in Patent Document 1 (for example, refer to Fig. 1).

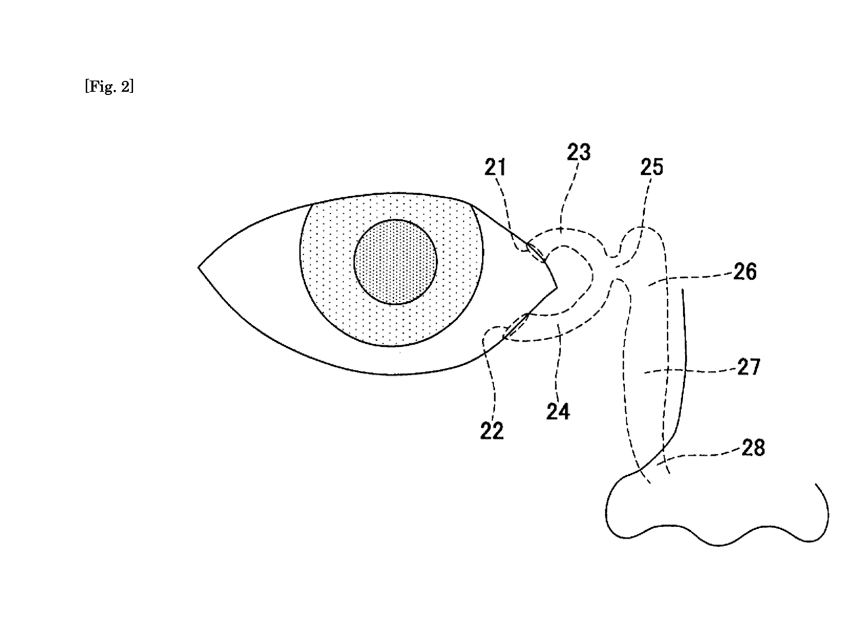

The nunchaku-shaped lacrimal duct tube includes a tube and a pair of bougies that is inserted from cuts at both sides of the tube, and the bougies are operated to guide the tube into a lacrimal duct and place the tube there. As shown in Fig. 2, a lacrimal duct is formed by lacrimal punctum (21 and 22), lacrimal canaliculus (23 and 24), a common canaliculus (25), lacrimal sac (26), a nasolacrimal duct (27), and others. The nunchaku-shaped lacrimal duct tube is inserted into the lacrimal duct.

However, the tube as shown in Patent Document 1 has a tip end of a blind-end structure, which causes a problem that a lacrimal fluid, a drug solution for verification of water passage at an examination, or the like stays in the lacrimal duct tube and is not discharged to the outside.

In addition, to insert the nunchaku-shaped lacrimal duct tube, it is necessary to fumble for intra-lacrimal duct operations. The bougies are blindly operated and thus may break through the tube or cut a hole at a site other than in the normal lacrimal tube (creating a false passage), which results in poor therapeutic outcomes.

As means for solving these problems, there has been suggested a lacrimal duct intubation instrument that is opened at a terminal end to allow discharge of a lacrimal fluid, drug solution, or the like, as described in Patent Document 2, for example. The lacrimal duct intubation instrument makes it possible to insert a lacrimal endoscope, instead of bougies, into the tube and guide the tube into a lacrimal duct while visually checking the status in the lacrimal duct.

In addition, the lacrimal duct intubation instrument described in Patent Document 2 has a reinforcement body in the vicinity of the terminal end of the tube to prevent that, at time of insertion, a bougie, lacrimal endoscope, or the like protrudes from the terminal end of the tube. The reinforcement body is illustrated in a ring shape in an embodiment, however, the ring shape is not intended to guide the bougies or the lacrimal endoscope into a hollow portion of the ring, and the tip end of the bougie or the like abuts against the end surface of the ring to prevent protrusion of the bougie or the like.

By arranging the thus structured reinforcement body, it can be expected that the tube is effective in preventing protrusion of the bougie or lacrimal endoscope from the tip end of the tube. However, the end surface of the reinforcement body is flat, and thus when the tip end of the bougie or the like abuts against the end surface of the reinforcement body, the tip end is prone to slide over the end surface of the reinforcement body. Accordingly, there is a fear that, if an excessive force acts on the lacrimal duct tube when the lacrimal duct tube is bent or guided into a obstructed lacrimal duct, the tip end of the bougie or the like slips sideways on the end surface of the reinforcement body, and the tip end of the bougie or the like breaks through the side surface of the tube, which results in damage to the lacrimal duct or creation of a false passage.

### Citation List

### Patent Literatures

Patent Document 1: Japanese Patent No. 2539325
Patent Document 2: International Publication WO 2011/049198

### Summary of Invention

### Technical Problem

In light of the foregoing circumstances, an object of the present invention is to provide a lacrimal duct tube that makes it possible to, if a tip end is opened, prevent that the tip end of an insertion aid such as a bougie or an endoscope projects from the opening of the tip end of the tube, and reduces the possibility that, when being inserted into a lacrimal duct, the tip end of an insertion aid such as a bougie or an endoscope breaks through the side surface of the tube.

### Solution to Problem

The inventors have earnestly conducted studies and found that the foregoing problems can be solved by configuring a lacrimal duct tube with an opening such that a stopper part including an inner wall surface to guide and stop the tip end of an insertion aid such as a bougie or an endoscope is inserted in the vicinity of the opening of the lacrimal duct tube, thereby completing the present invention.

Specifically, the gist of the present invention is as follows:
[1] A lacrimal duct tube, including an integrated tube that is placed in a lacrimal duct and capable of insertion of an insertion aid in a detachable manner, wherein the tube has an opening at a terminal end, and in the vicinity of the opening, a stopper part for stopping the insertion aid is inserted in the tube, and the stopper part is opened at both ends in an axial direction of the tube and has a cylindrical structure including an inner wall surface to guide and stop the insertion aid.
[2] The lacrimal duct tube according to [1], wherein the inner wall surface of the stopper part includes a tapered portion that continuously decreases in width in a direction perpendicular to the tube axial direction toward a terminal end side of the tube.
[3] The lacrimal duct tube according to [1], wherein the inner wall surface of the stopper part includes a stepped portion that decreases stepwise in width in the direction perpendicular to the tube axial direction toward the terminal end side of the tube.
[4] The lacrimal duct tube according to [1], wherein the inner wall surface of the stopper part includes a protruded portion toward an opposed inner wall surface.

### Advantageous Effects of Invention

According to the lacrimal duct tube according to the present invention, when the lacrimal duct tube is inserted into a lacrimal duct using an insertion aid, the tip end portion of the insertion aid is guided to and stopped at the stopper part with a cylindrical structure in the lacrimal duct tube, and thus the insertion aid does not greatly slip sideways when abutting against the stopper part, and it is possible to effectively reduce the possibility that the insertion aid protrudes from the leading end of the lacrimal duct tube or breaks through the side surface of the tube.

### Brief Description of the Drawings

Fig. 1 is a schematic view of a conventional typical nunchaku-shaped lacrimal duct tube;
Fig. 2 is an illustrative diagram showing an anatomical structure of a lacrimal duct; and
Fig. 3(a) is a cross-sectional view of one example of a placement portion of the lacrimal duct tube of the present invention, Fig. 3(b) is a cross-sectional view of a tip end portion of the lacrimal duct tube into which an insertion aid is inserted in the example shown in Fig. 3(a), Fig. 3(c) is a cross-sectional view of another example of the tip end portion of the placement portion of the lacrimal duct tube of the present invention, and Fig. 3(d) is a cross-sectional view of another example of the tip end portion of the placement portion of the lacrimal duct tube of the present invention.

### Description of Embodiments

The present invention will be described below in more detail.

Lacrimal duct referred to in the present invention is a duct (ocular adnexa) composed of upper/lower lacrimal punctum (21/22), upper/lower lacrimal canaliculus (23/24), a common canaliculus (25), a lacrimal sac (26), a nasolacrimal duct (27), a nasal cavity (not shown), and Hasner's valve (not shown), as shown in Fig. 2, and configured to guide a lacrimal liquid produced by a lacrimal gland (not shown) from an eye surface to an inferior nasal meatus (28). Fig. 2 shows schematically an anatomical structure of a lacrimal duct. In addition, a duct extending from the upper lacrimal punctum (21) through the upper lacrimal canaliculus (23), and the common canaliculus (25) to the inferior nasal meatus (28) is referred to as an upper lacrimal duct, and a duct extending from the lower lacrimal punctum (22) through the lower lacrimal canaliculus (24), and the common canaliculus (25) to the inferior nasal meatus (28) is referred to as a lower lacrimal duct.

The lacrimal duct tube of the present invention is formed from an integrated tube that is placed in the lacrimal duct. The term "integrated" means that two tubes to be respectively inserted into the upper and lower lacrimal ducts are integrated into one. The integrated tube refers to a soft intubation instrument with a predetermined length that is inserted into a lacrimal duct for reconstructive treatment of the lacrimal duct.

The integrated tube has openings at both terminal ends thereof. The provision of the openings in the tube allows securing of a view field of a lacrimal endoscope and execution of a water passage investigation through the openings.

The integrated tube may be structured to have a cylindrical shape, for example, that has openings formed at both terminal ends and a hollow portion to establish communication between these openings. The cylindrical shape may have almost uniform inner and outer diameters over the entire length or may have an almost uniform small-diameter portion at a central part.

As another example of a structure of the integrated tube, the integrated tube may include a cylindrical shape that has a rod-shaped portion central part, openings at both terminal ends thereof, and a hollow portion communicating with the openings. In this case, the integrated tube may have an almost uniform outer diameter over the entire length or may have an almost uniform small-diameter portion at the rod-shaped central part.

Of the foregoing examples of the integrated tubes, the tube having an almost uniform small-diameter portion at the central part may have, more specifically, cylindrical portions on the both sides to be placed from a lacrimal sac to a nasolacrimal duct and have a rod-shaped portion to be placed from a lacrimal punctum to a lacrimal canaliculus. The lacrimal duct intubation instrument having the shape and structure as described above and the central part softer than the both end portions is called nunchaku-shaped lacrimal duct tube as described above.

The nunchaku-shaped lacrimal duct tube is named as such for similarity in shape to a nunchaku for use in a Chinese martial art. The nunchaku-shaped lacrimal duct tube is preferably soft at the central part to an extent that the tube is inverted U-shaped when being lifted with a central point along its length as a supported point.

There is no particular limitation on shape of the both terminal ends of the integrated tube. From the viewpoint of prevention of damage to a lacrimal duct, the both terminal ends of the integrated tube preferably have an edge-less shape. For example, the external shape of the terminal end portions may be a shape other than pointed shapes such as an approximately conical shape and a pyramid shape, a shape chamfered at end surfaces, or a round-cornered shape, but is not limited to these shapes.

The insertion aid in the present invention refers to an instrument that is configured to, for insertion and placement of the lacrimal duct tube into the lacrimal duct, be inserted into the lacrimal duct tube to guide the lacrimal duct tube into the lacrimal duct, and then removed from the lacrimal duct tube. For example, bougies apply to the insertion aid. In addition, to insert the lacrimal duct tube into a lacrimal duct while visually observing the inside of the lacrimal duct, a lacrimal endoscope is used instead of bougies. The lacrimal endoscope also applies to the insertion aid.

In the present invention, the integrated tube may be provided with an insertion part into which a bougie or a lacrimal endoscope is inserted. In particular, as for the so-called nunchaku-shaped lacrimal duct tube, it is generally not possible to insert a bougie or a lacrimal endoscope into the central part of the tube in many cases. Therefore, to insert a lacrimal endoscope into the hollow portions of the cylindrical shapes on the both sides of the central part, it is necessary to provide an insertion part to establish communication between the outside and the hollow portion. The insertion part may be provided at side walls or the like of the cylindrical portions. There is no particular limitation on structure of the insertion part, and thus the insertion part may be selected as appropriate from among a small hole, cut, and the like.

There is no particular limitation on constitutional material (ingredient) for the integrated tube. For example, the material may be silicone, polyurethane, isobutylene copolymer, and resin composite containing an alloy of these ingredients and the like, but is not limited to these materials. In the present invention, there is no particular limitation on the foregoing alloy. For example, in the case of using an alloy of a thermoplastic polyurethane resin and isobutylene copolymer, the hardness of the tube can be adjusted by changing the ratio between the isobutylene copolymer (A) and the thermoplastic polyurethane resin (B). The larger the ratio of the thermoplastic polyurethane resin (B), the harder the tube becomes. From the viewpoint of antithrombogenicity, surface slidability, and flexibility, the tube preferably contains the isobutylene copolymer (A) of 1% by weight or more (that is, the ratio between the isobutylene copolymer (A) and the thermoplastic polyurethane resin (B) is (A)/(B) = 1/99 to 99/1 by weight). Above all, from the viewpoint of wear resistance, the ratio between the isobutylene copolymer (A) and the thermoplastic polyurethane resin (B) is preferably (A)/(B) = 1/99 to 70/30 by weight. In particular, from the viewpoint of compression stress, the ratio between the isobutylene copolymer (A) and the thermoplastic polyurethane resin (B) is preferably (A)/(B) = 1/99 to 50/50 by weight. The resin composite for the integrated tube for use in the present invention may be composed of only the isobutylene copolymer (A) and the thermoplastic polyurethane resin (B), or may be mixed with other ingredients.

Preferred as the isobutylene copolymer (A) is "SIBSTAR (registered trademark) 102T" produced by Kaneka Corporation, which is styrene-isobutylene-styrene block copolymer (hereinafter, also referred to as SIBS). Preferred as the thermoplastic polyurethane resin (B) (hereinafter, also referred to as TPU) are "Miractran E385PNAT" produced by Nippon Miractran Co., Ltd. and "Tekotan TT1074A" produced by Lubrizol Corporation, which are ether aromatic cyclic polyurethanes, or "Tecoflex EG100A" and "Tecoflex EG85A" produced by Lubrizol Corporation, which are ether cycloaliphatic polyurethanes, or "Karubotan PC3575A" produced by Lubrizol Corporation, which is a polycarbonate-based polyurethane.

In the present invention, in the vicinity of the opening at a terminal end of the integrated tube, a stopper part is inserted into the tube. When openings are formed at both terminal ends of the integrated tube, the stopper part may be inserted into at least one of the two openings. That is, the stopper part may be inserted in the vicinity of one of the openings in the integrated tubes, or may be inserted in the vicinity of the openings in the integrated tube at the both terminal ends. For example, in the case of placing the lacrimal duct tube through only one of the upper/lower lacrimal canaliculus or inserting and placing the lacrimal duct tube from the common canaliculus due to scarring of the upper/lower lacrimal canaliculus, the stopper part may be inserted in the vicinity of one of the openings formed at the both terminal ends of the integrated tube. In the case of placing one lacrimal duct tube through both of the upper/lower lacrimal canaliculus, the stopper part may be inserted in the vicinities of the openings formed at the both terminal ends of the integrated tube. In the former case, an insertion part for inserting an endoscope or the like may be configured to be inserted through the opening at which the stopper part is not arranged. In addition, in the case of inserting the stopper part in the vicinities of the openings formed at the both terminal ends of the integrated tube, it is possible to easily place the tube in the upper/lower lacrimal canaliculus using one lacrimal duct tube in conjunction with arrangement position of the stopper part to be described later.

The stopper part needs to be shaped at least such that the stopper part can be arranged in the vicinity of the opening of the integrated tube to act as a stopper for an insertion aid, as well as can include an inner wall surface to guide and stop the insertion aid. Further, when the stopper part and the tube are arranged coaxially in general, the stopper part has a cylindrical structure opened at both ends in its axial direction (which coincides with the axial direction of the tube). This enables discharge of a lacrimal fluid, drug solution, or the like from the tube. Accordingly, when an endoscope is used as an insertion aid, it is possible to ensure the field of view of the endoscope.

Furthermore, the cylindrical structure of the stopper part is arranged coaxially with the tube and is configured to, when inserting the tube into the lacrimal duct, prevent the insertion aid from protruding from the tip end and side wall of the tube, and stop the tip end of the insertion aid on the inner wall surface of the cylindrical structure.

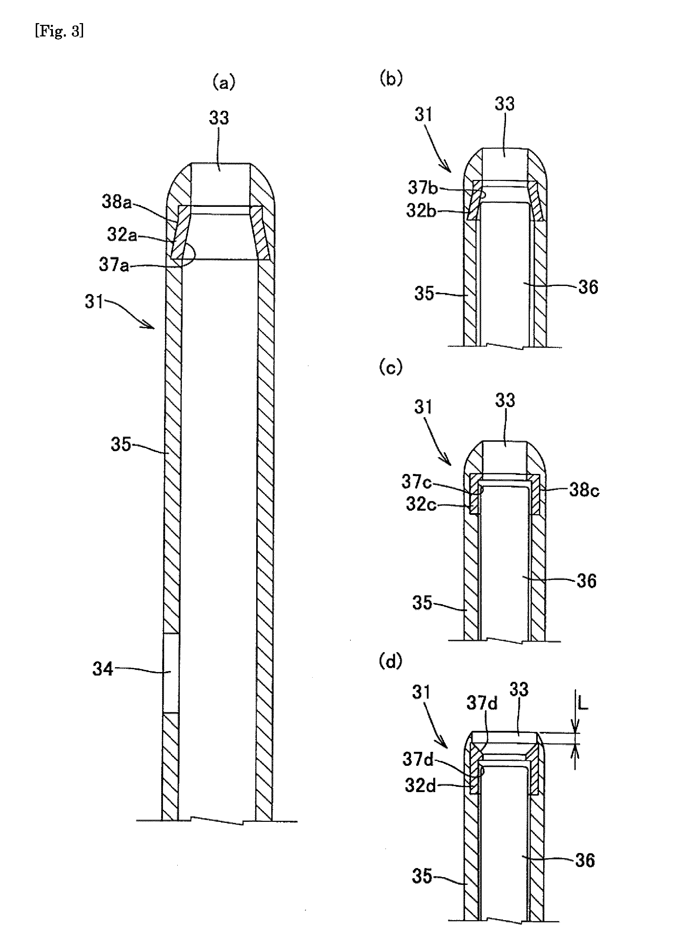

There is no particular limitation on the foregoing structure. For example, the stopper part is preferably structured such that its width perpendicular to the axial direction of the stopper part and the tube decreases continuously from the base end side to the terminal end side of the tube. In such a structure, an abutment surface between the stopper part and the insertion aid is small and the width of the tube at the base end side is larger, which may produce the effect of allowing the insertion aid to be relatively easily removed from the lacrimal duct tube. Such a structure preferably has a shape as shown in Fig. 3(b), for example, in which the surface perpendicular to the axial direction becomes smaller in diameter from the base end side to the terminal end side, that is, a so-called tapered shape. Accordingly, a bougie or an endoscope is sandwiched in the stopper part, which makes it possible to prevent that the bougie or the endoscope breaks through the tube. In the example of Fig. 3(b), the inner wall surface is inclined in its cross section at a predetermined angle. However, the inner wall surface is not limited to this, and the inner wall surface may be structured such that its diameter becomes smaller in a bowl shape with continuous changes in the inclination angle, for example. In addition, it is possible to employ a structure in which the width of the inner wall surface decreases continuously or a structure in which the inner wall surface continuously reduces in diameter, within the scope of advantages of the present invention.

As another structure, the stopper part is also desirably structured so as to be arranged coaxially with the tube and include a stepped portion with which the width of the stopper part perpendicular to the axial direction of the stopper part and the tube decreases stepwise. In such a structure, the inner wall surface up to the stepped portion and the side wall surface of the insertion aid are parallel to each other, whereby the insertion aid tends to be stable when being guided into the stopper part. Further, when the stepped portion is disposed in the vicinity of the terminal end side of the tube, the distance from the stepped portion to the tube terminal end can be shortened. Thus, it is possible to, when an endoscope is used as an insertion aid, ensure a wider field of view of the endoscope. Such a structure may include a stepped portion at the terminal end side of the tube as shown in the cross-sectional view of Fig. 3(c), for example. By such a structure, it is possible to, when the lacrimal duct tube is inserted using an insertion aid, reduce the risk that the tip end of the insertion aid abuts against and stop on the stepped portion of the stopper part and thus the insertion aid protrudes from the tip end of the tube or breaks through the side wall of the tube. In the example of Fig. 3(c), the stepped portion of the stopper part is arranged at its terminal end side (upper side of the drawing). However, the stepped portion is not limited to this, and for example, the stepped portion may be arranged at the intermediate portion of the stopper part along its entire axial length, or may be arranged at any other position. The number of the stepped portion is not limited to only one but may be two or more.

As still another structure, the stopper part is also preferably structured to have at a predetermined position on the inner wall surface a protruded portion that protrudes toward the opposed inner wall surface. The protruded portion may be arranged on a portion of the inner wall surface in the circumferential direction or may be arranged around the entire circumference of the inner wall surface. There is no particular limitation on position of the protruded portion, provided that the protruded portion is arranged at a position that can guide an insertion aid to the inside of the stopper part, and the protruded portion may be arranged at any position in the axial direction of the tube (axial direction of the stopper part). For example, the protruded portion may be arranged at the terminal end on the tube terminal end side in the axial direction, or may be arranged at an intermediate position along the entire axial length of the stopper part, or may be arranged at any other position.

As still another structure, the stopper part is also preferably structured as shown in the cross-sectional view of Figure 3(d) to have a tapered shape at the terminal end of the stopper part shown in Fig. 3(c) in which the inner wall surface increases in diameter from the base end side to the terminal end side. This makes it possible to, when an endoscope is inserted as an insertion aid, ensure a wide field of view of the endoscope.

The arrangement position of the stopper part is preferably set in the vicinity of the opening of the tube terminal end and at a predetermined distance from the opening (endmost portion of the opening). When a lacrimal endoscope is used as an insertion aid, the predetermined distance is decided from the viewpoints of serving as a stopper for a lacrimal endoscope and ensuring the field of view of the lacrimal endoscope. To ensure the field of view of the endoscope, the predetermined distance is preferably shorter. Taking into account a relation with the outer diameter of the endoscope, the length of the predetermined distance is preferably set to two times or less the outer diameter of the endoscope.

The scope of field of view of a lacrimal endoscope may be affected by the size of the diameter of the opening (in particular, diameter of the terminal end of the opening), aside from a so-called viewing angle of the lacrimal endoscope. The diameter of the opening (also referred to as opening diameter) is preferably larger from the viewpoint of ensuring the field of view of the lacrimal endoscope. Meanwhile, when the opening diameter increases, the tube becomes thinner at the terminal end portion, which makes it difficult to hold the stopper part. In this case, when the tube is inserted into the lacrimal duct using a force applied to the lacrimal endoscope, the stopper part may pass through the opening. Thus, when a lacrimal endoscope is used as an insertion aid, if the outer diameter of the tube is 1.1 to 2.0 mm, for example, the diameter of the opening (opening diameter) is preferably 0.6 to 1.0 mm, more preferably 0.7 to 0.9 mm, from the viewpoints of holding the stopper part, preventing passage of the stopper part, and ensuring the field of view of the lacrimal endoscope. When no endoscope is used as an insertion aid, the diameter of the opening may be decided with consideration given to discharge of a lacrimal fluid, drug solution, or the like, water passage property, or the like.

As described above, by arranging the stopper part in the vicinity of the terminal end opening of the tube and at a predetermined distance from the opening, it is possible to smoothly insert the tube into the lacrimal duct using a force applied to the lacrimal endoscope while ensuring the field of view of the lacrimal endoscope. Further, the stopper part serves as a stopper to reduce a risk of accidental passage of the lacrimal endoscope through the opening. Such an effect can be further improved by adjusting the opening diameter of the opening as described above as well as adjusting the distance from the opening. In addition, when the lacrimal endoscope is used, it is possible to surely know the route through which the tube is passed, which avoids a problem that the tube creates a false passage resulting in damage to mucous membranes with bleeding or the like. Further, the vicinity of the tube terminal end portion is reinforced by the stopper part, which makes it possible to perform an opening process even in post-processing.

There is no particular limitation on constitutional material for the stopper part. From the viewpoint of guiding and stopping an insertion aid, hard material is preferred, for example, including various hard resins, metal such as stainless, and the like. From the viewpoint of preventing corrosion due to contact with body fluids, drug solutions, or the like, stainless steel is preferred.

In order to enhance insertability of a lacrimal duct intubation instrument into the lacrimal duct, hydrophilic coating may be provided on the outside of the tube. The coating produces lubricity upon contact with blood, which reduces resistance of insertion. There is no particular limitation on kind of hydrophilic coating, and hydrophilic polymers such as poly(2-hydroxyethyl methacrylate), polyacrylamide, polyvinylpyrrolidone, and polyethylene glycol, or blends thereof can be preferably used.

The lacrimal duct tube according to the present invention will be described with reference to one embodiment shown in the drawings. However, the present invention is not limited to the embodiment.

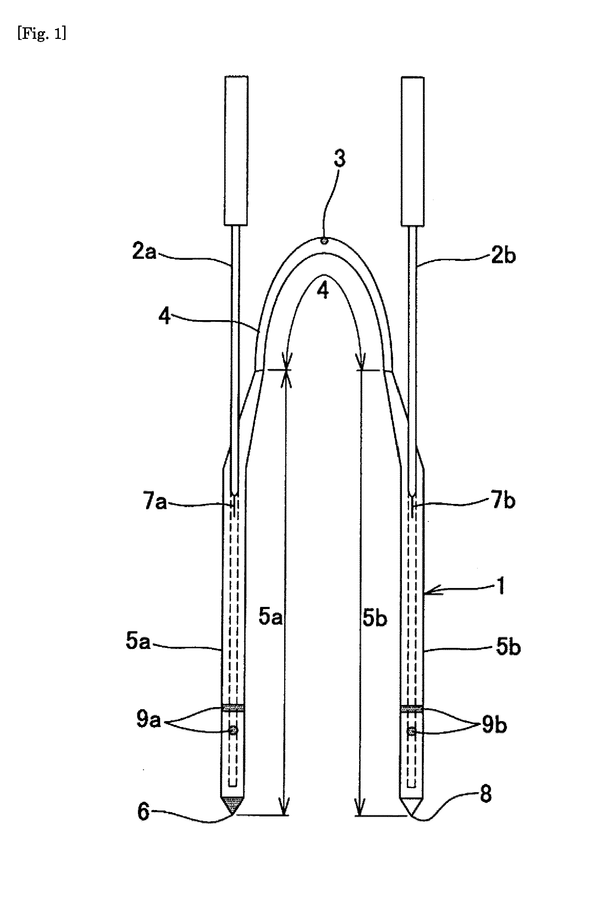

Fig. 1 shows one example of a conventional typical nunchaku-shaped lacrimal duct tube (in which bougies are inserted). Fig. 3(a) is a schematic cross-sectional view of one embodiment of a tube part of the lacrimal duct tube in the present invention that is placed in an inferior nasal meatus and the like in a lacrimal duct. Fig. 3(b) is a schematic cross-sectional view of a terminal end portion of the tube part, illustrating the state in which a lacrimal endoscope is inserted into a hollow portion of the tube part in the example shown in Fig. 3(a). Fig. 3(c) is a schematic cross-sectional view of another example of the terminal end portion of the tube part. Fig. 3(c) shows the state in which a lacrimal endoscope is inserted into the hollow portion of the tube part as in Fig. 3(b).

First, the conventional typical nunchaku-shaped lacrimal duct tube shown in Fig. 1 will be described. The conventional typical nunchaku-shaped lacrimal duct tube 1 includes: a cylindrical or rod-shaped central part 4 that is placed in lacrimal punctum, lacrimal canaliculus, common canaliculus, and lacrimal sac; a first cylindrical part 5a that is connected to one end of the central part 4 and is placed in a lacrimal sac, nasolacrimal duct, Hasner's valve, and inferior nasal meatus; and a second cylindrical part 5b that is connected to the other end of the central part 4 and is placed in a lacrimal sac, nasolacrimal duct, Hasner's valve, and inferior nasal meatus. The central part 4 has a so-called nunchaku shape that is made thinner than the first cylindrical part 5a and the second cylindrical part 5b.

First terminal end 6 of the first cylindrical part 5a and a second terminal end 8 of the second cylindrical part 5b are blind ends and have pointed tips. The tip end portion including the first terminal end 6 is colored to allow an operator to easily discriminate from the tip end portion including the second terminal end 8. In addition, two marks 9a are given to the first cylindrical part 5a at predetermined positions from the first terminal end 6, which allows an operator to visually check the depth of insertion. Similarly, marks 9b are given to the second cylindrical part 5b.

The first cylindrical part 5a and the second cylindrical part 5b have on side walls cuts 7a and 7b for insertion of bougies, respectively, in the vicinity of the central part 4. Through the cuts 7a and cuts 7b, a first bougie 2a and a second bougie 2b as insertion aids are inserted into hollow portions of the first cylindrical part 5a and the second cylindrical part 5b, respectively.

The central part 4 has a midpoint 3 provided on the approximate center of the nunchaku-shaped lacrimal duct tube to facilitate verification of the insertion position or the placement position.

The basic structure of the lacrimal duct tube according to the present invention is similar to that of the nunchaku-shaped lacrimal duct tube shown in Fig 1. There is a difference between the two in the structures of the first cylindrical part 5a and the second cylindrical part 5b. Thus, the difference will be described below. Configurations except for the difference, for example, provision of the marks 9a (9b) shown in Fig. 1, coloring of the terminal end portion, and the like may be employed in the lacrimal duct tube according to the present invention.

A lacrimal duct tube as one embodiment of the present invention is formed by applying cylindrical tubes as shown in Fig. 3(a), instead of the first cylindrical part 5a and the second cylindrical part 5b, to the conventional nunchaku-shaped lacrimal duct tube shown in Fig. 1. Specifically, the tube shown in Fig. 3(a) is connected to both ends of the central part 4 shown in Fig. 1 in accordance with the determined method, thereby to form a lacrimal duct tube including an integrated tube according to the present invention. Fig. 3(a) is a cross-sectional view of one cylindrical tube of the integrated tube. The other tube is virtually the same in structure as the one tube, and thus descriptions thereof will be omitted.

As shown in Fig. 3(a), in a lacrimal duct tube 31 as one embodiment of the present invention, a cylindrical part 35 has a terminal end opening 33 at a terminal end thereof, and in the vicinity of the terminal end opening 33, a stopper part 32a is inserted at a predetermined position from the terminal end opening 33, coaxially with the cylindrical part 35. In addition, a cut 34 is provided on a side wall in proximity to the center of the cylindrical part 35 to establish communication between the outside and the hollow portion. Such an insertion aid as a bougie, lacrimal endoscope, or the like can be inserted into the cut 34.

In this example, the stopper part 32a has a cylindrical shape opened at both axial terminal ends. An inner wall surface 37a and an outer wall surface 38a form a structure that continuously reduces in diameter at a predetermined constant taper angle in increasing proximity to the terminal end side (upper side of the drawing) of the cylindrical part 35. The stopper part 32a also has an inner wall surface parallel to the axial direction of the cylindrical part 35 at the terminal end side of the inner wall surface 37a. The base end side (lower side of the drawing) of the inner wall surface 37a may be continued and aligned with the inner wall surface of the cylindrical part 35. Thus, in this example, a concave portion corresponding to the cylindrical structure of the stopper part 32a is formed in the vicinity of the terminal end opening 33 of the cylindrical part 35, and the stopper part 32a is inserted into the concave portion and held at the cylindrical part 35.

Further, in this example, the inner diameter of the opening of the stopper part 32a at the terminal end side (upper side of the drawing) is equal to the inner diameter of the terminal end opening 33 of the cylindrical part 35. Alternatively, the inner diameter of the terminal end opening 33 may be smaller than the inner diameter of the opening of the stopper part 32a (not shown). This makes it possible to fix the stopper part 32a more firmly to the inside of the cylindrical part 35 and make the stopper part 32a less prone to pass through the terminal end opening 33. However, when an endoscope is used as an insertion aid, it is preferred to avoid hindrance to the field of view of the endoscope as much as possible. For example, the inner diameter of the terminal end opening 33 of the cylindrical part 35 may be slightly smaller than the inner diameter of the terminal end side of the stopper part 32a.

As shown in Fig. 3(b), in this embodiment, a stopper part 32b is a cylindrical in shape, has an inner wall surface 37 where an insertion aid 36 such as a bougie and an endoscope is guided and a tip thereof is stopped, and has a predetermined tapered shape in which an inner wall surface 37b continuously reduces in diameter from the base end side (lower side of the drawing) to the tip end side (upper side of the drawing). Thus, since the tip end portion of the insertion aid 36 is disposed in the inside of a cylindrical stopper part 32b, it is possible to effectively reduce the possibility that, when the lacrimal duct tube is inserted into the lacrimal duct using the insertion aid 36, the insertion aid 36 slips sideways on the abutment surface of the stopper part and breaks through the side wall of the cylindrical part 35. In addition, since the inner wall surface has a predetermined tapered shape, contact area between the inner wall surface of the stopper part 36 and the insertion aid 36 is relatively small, and thus it can be expected that, when the insertion aid 36 is removed, the removal is relatively easy without a hitch against the stopper part 36.

Fig. 3(c) shows another example of a structure of the stopper part in the lacrimal duct tube 31 as one embodiment of the present invention. In this example, although the structure of the stopper part is different from that shown in Fig. 3(b), the lacrimal duct tube 31 is the same as that in the foregoing example.

In this example, a stopper part 32c has a cylindrical shape opened at both axial terminal ends, and an outer wall surface 38c is formed parallel to the axial direction of the cylindrical part 35. An inner wall surface 37c includes: a first-step inner wall surface parallel to the axial direction of the cylindrical part 35; a second-step inner wall surface that is smaller in diameter than the inner diameter of the first-step inner wall surface, is parallel to the axial direction of the cylindrical part 35, and is positioned on the further terminal end side than the first-step inner wall surface; and an inner wall surface that is continued to these inner wall surfaces and is parallel to a direction perpendicular to the axial direction of the cylindrical part 35. The inner wall surface parallel to the direction perpendicular to the axial direction of the cylindrical part 35 forms a stepped portion, and the tip end of the insertion aid 36 abuts against and is locked (stopped) on the inner wall surface. In addition, the insertion aid 36 is guided to the first-step inner wall surface.

As described above, since the tip end portion of the insertion aid 36 is disposed in the inside of the cylindrical stopper part 32c, it is possible to effectively decrease the possibility that, when the lacrimal duct tube is inserted into the lacrimal duct using the insertion aid 36, the insertion aid 36 slips sideways on the abutment surface of the stopper part and breaks through the side wall of the cylindrical part 35. In addition, by using the stopper part 32c in this example, the first-step inner wall surface up to the stepped portion and the side wall surface of the insertion aid 36 are made parallel to each other, whereby the insertion aid 36 tends to be stable when being guided to the inside of the stopper part 32c. Further, since the stepped portion is disposed at a position near the terminal end side of the cylindrical part 35, the distance from the stepped portion to the terminal end of the opening 33 of the cylindrical part 35 can be shortened. Thus, it is possible to ensure a wider field of view in the case of using an endoscope as an insertion aid.

Also in this example, the inner diameter of the opening of the stopper part 32c at the terminal end side (upper side of the drawing) may be equal to the inner diameter of the terminal end opening 33 of the cylindrical part 35, as in the case of Fig. 3(a), or the inner diameter of the terminal end opening 33 may be smaller (not shown). In the latter case, it is possible to fix the stopper part 32c more firmly to the inside of the cylindrical part 35 and make the stopper part 32c less prone to pass through the terminal end opening 33. However, if an endoscope is used as an insertion aid, it is preferred to avoid hindrance to the field of view of the endoscope as much as possible. For example, the inner diameter of the terminal end opening 33 of the cylindrical part 35 may be slightly smaller than the inner diameter of the stopper part 32c at the terminal end side.

Fig. 3(d) shows another example of a structure of the stopper part in the lacrimal duct tube as one embodiment of the present invention. In this example, the structure of Fig. 3(c) further has a tapered shape in which an inner surface wall 37d of a stopper part 32d at the terminal end continued from the second-step inner wall surface increases in diameter from the base end side to the terminal end side. In this example, a stepped portion shown in Fig. 3(c) serves as a stepped portion and a protruded portion in the present invention.

In this example, by shortening length L of the foregoing predetermined distance, it is possible to, when an endoscope is used as an insertion aid, bring the lens of the endoscope closer to the tube terminal end, thereby ensuring a wider field of view of the endoscope. In addition, in this example, the inner diameter of the opening of the stopper part 32d at the terminal end side (upper side of the drawing) may be equal to the inner diameter of the terminal end opening 33 of the cylindrical part 35 as shown in Fig. 3(d). Alternatively, in order to allow the stopper part 32d to be fixed more firmly to the cylindrical part 35 and make the stopper part 32d less prone to pass through the terminal end opening 33 as described above, the inner diameter of the terminal end opening 33 of the cylindrical part 35 may be slightly smaller than the inner diameter of the opening of the stopper part 32d at the terminal end side (upper side of the drawing) (not shown). Accordingly, it is possible to, when an endoscope is used as an insertion aid, ensure the field of view of the endoscope.

### Reference Signs List

- 1: Lacrimal duct intubation instrument (nunchaku-shaped lacrimal duct tube)
- 2a: First bougie
- 2b: Second bougie
- 3: Midpoint
- 4: Central part
- 5a: First cylindrical part
- 5b: Second cylindrical part
- 6: First terminal end
- 7: Cut for insertion of bougies
- 8: Second terminal end
- 21: Upper lacrimal punctum
- 22: Lower lacrimal punctum
- 23: Upper lacrimal canaliculus
- 24: Lower lacrimal canaliculus
- 25: Common canaliculus
- 26: Lacrimal sac
- 27: Nasolacrimal duct
- 28: Inferior nasal meatus
- 31: Lacrimal duct tube
- 32a: Stopper part
- 32b: Stopper part
- 32c: Stopper part
- 32d: Stopper part
- 33: Terminal end opening
- 34: Cut
- 35: Cylindrical part
- 36: Insertion aid
- 37a: Inner wall surface
- 37b: Inner wall surface
- 37c: Inner wall surface
- 37d: Inner wall surface
- 38a: Outer wall surface
- 38c: Outer wall surface
- L: Predetermined distance

## Claims

1. A lacrimal duct tube, comprising an integrated tube that is placed in a lacrimal duct and capable of insertion of an insertion aid in a detachable manner, wherein
the tube has an opening at a terminal end,
in the vicinity of the opening, a stopper part for stopping the insertion aid is inserted in the tube, and
the stopper part is opened at both ends in an axial direction of the tube and has a cylindrical structure including an inner wall surface to guide and stop the insertion aid.

2. The lacrimal duct tube according to Claim 1, wherein the inner wall surface of the stopper part includes a tapered portion that continuously decreases in width in a direction perpendicular to the tube axial direction toward a terminal end side of the tube.

3. The lacrimal duct tube according to Claim 1, wherein the inner wall surface of the stopper part includes a stepped portion that decreases stepwise in width in the direction perpendicular to the tube axial direction toward the terminal end side of the tube.

4. The lacrimal duct tube according to Claim 1, wherein the inner wall surface of the stopper part includes a protruded portion toward an opposed inner wall surface.
